# EUROPEAN PATENT APPLICATION

(11) **EP 2 316 436 A1**
(43) Date of publication of application: **04.05.2011**
(21) Application number: 09013681.3
(22) Date of filing: 30.10.2009
(51) Int. Cl.: A61K 9/70, A61P 17/12, A61K 38/43

(54) **Tape, in particular adhesive tape, for the treatment of skin disorders comprising a film , in particular dissolvable film, containing at least one enzyme**

(71) Applicant: Johnson & Johnson GmbH, 41470 Neuss (DE)
(72) Inventor: Perez, Encarna, 10243 Berlin (DE)
(74) Representative: Metten, Karl-Heinz

(57) **Abstract**

The present invention relates to a tape, in particular adhesive tape, for the treatment of skin disorders comprising at least one base layer having a first surface facing away from the skin during use and a second surface facing towards the skin during use, and at least one first film, in particular dissolvable film, on the side of said second surface, which covers at least part of the second surface of said base layer, said first film, in particular dissolvable film, having a first surface facing away from the skin during use and a second surface facing towards the skin during use and comprising at least one enzyme, in particular a proteolytic enzyme.

## Description

The present invention relates to a tape, in particular adhesive tape, for the treatment of skin disorders, such as hyperkeratotic skin diseases, comprising a film, in particular dissolvable film, containing at least one enzyme.

Hyperkeratotic tissues such as corns and calluses are well defined, thickened lesions of the epidermis. These tissues usually occur at those skin sites which experience chronic mechanical stress. Hyperkeratotic tissues may cause debilitating pain. For example, corns and calluses will stop people from wearing those shoes they would like to wear. Such skin disorders will thus have a dramatic effect on entire life style choices.

A variety of attempts have been made to treat, medicate or cure skin disorders such as hyperkeratotic tissues.

According to US 4,895,727 maximum effective treatment of localized skin disease conditions shall be accomplished with a topical formulation containing a pharmacologically active agent such as wrinkle reducing agents and a water-soluble zinc containing compound.

According to US 5,213,978 an isolated enzyme material' having protease activity obtained from *Micrococcus sedentarius* shall be usable in pharmaceutically acceptable formulations to treat corns and calluses on the feet.

In WO 87/04617 A1 a composition for the treatment of hyperkeratotic skin diseases, sebor-rheic eczema, dermatomycosis and onychomycosis, and thickened and chapped skin is disclosed which comprises propylene glycol and/or polyethylene glycol and urea as active main components. In these compositions urea has to be present in amounts of 5 to 20 weight percent and propylene glycol and/or polyethylene glycol have to be present in amounts of 40 to 80 weight percent. Optionally, active substances and additives such as glycerol, gel forming agents, C₁₋₄-a1cohols, glucokorticoids, and antimycotic substances may be present.

US 6,569,437 B1 describes a method for treating at least one of an abnormal skin condition, disease, disorder and for regulating the effects of skin atrophy and for improving the texture and appearance of the skin which comprises topically administering on an area of the skin an effective amount of a composition which comprises an acid protease which is enzymatically active below pH 5.5, and an acidic buffer which lowers the surface pH of the skin to below about pH 5.5 for a period of time effective for the treatment of at least one condition, disease, and disorder of the skin. Suitable acidic proteases can be selected from fungal proteases, bacterial proteases, mammalian proteases and mixtures thereof.

A fast acting corn and callus remover shall according to WO 96/17601 A1 be obtained by applying a composition comprising a 4-carbon-α-ketoacid in a range of about 2 to about 64 weight percent and a pharmaceutically acceptable carrier material such as propylene glycol, petrolatum, ethanol, acetone, dimethylsulphoxide, pad devices, discs, plaster and flexible col-lodion.

US 6,471,986 B1 is about a medicated pad comprising a center cushion layer having adhesive and liquid absorbing properties, a medicated plaster secured to the underside of said center cushion layer, a barrier layer interposed between said medicated plaster and said center cushion layer, an outer layer secured to said center cushion layer, and a layer of adhesive material on the underside of said outer layer. Said central cushion layer has to be formed from a hydrocolloid which is a natural or synthetic rubber co-mingled with a cellulosic or hygroscopic material or wherein said cushion layer is formed from a hygroscopic hydrocolloid co-mingled with a solid matrix of rubber. With the barrier layer diffusion of any medicament in said medicated plaster to said center cushion layer shall be prevented. A suitable medicament included in the medicated plaster comprises salicylic acid. With the aforementioned medical pad warts, corns and calluses shall be removable.

US 5,547,989 discloses a topical composition for the treatment of corns and calluses. Said topical composition has to be in gel form and has to essentially consist i) of 7 to 13 carbon dicarboxylic acids or corresponding salts or esters thereof in a range of about 4 to about 64 wt.-% and ii) of a pharmaceutically acceptable carrier material. Azelaic acid is mentioned to be a preferred dicarboxylic acid. Suitable pharmaceutically acceptable carrier materials shall comprise polyethylene glycol 1000 isocetylether, cellulose, and 2-hydroxypropylether.

US 6,858,215 B2 is about a composition for treatment of hyperkeratotic mammalian tissue which comprises at least one food-grade proteolytic enzyme and a pharmaceutically or cosmetically acceptable carrier. Said at least one food-grade proteolytic enzyme comprises Subtilisin Carlsberg or a fungal protease/peptidase complex produced by submerged fermentation of selected strains of *Aspergillus oryzae.* Formulations tested on affected skin were made from proteolytic enzymes and petrolatum as the carrier material.

WO 2004/009050 A1 describes an orally consumable film for delivering breath freshening agents to the oral cavity. The film compositions which dissolve or disintegrate rapidly when applied in the oral cavity shall be comprised of a homogenous mixture of an enzyme and a water-soluble or water-dispersible film forming polymer.

There still has been a need for improved tapes for the treatment of skin disorders, in particular skin disorders relating to hyperkeratotic tissues, such as corns, warts or calluses. It, therefore, has been an object of the present invention to provide tapes which are rather comfortable for the user and yet offer a quick remedy for skin disorders such as corns, calluses and warts, in particular while simultaneously providing for significant pain release.

Accordingly, a tape, in particular adhesive tape, for the treatment of skin disorders has been found comprising at least one base layer having a first surface facing away from the skin during use and a second surface facing towards the skin during use, and at least one first film, in particular dissolvable film, on the side of said second surface, which covers at least part of the second surface of said base layer, said first film, in particular dissolvable film, having a first surface facing away from the skin during use and a second surface facing towards the skin during use and comprising at least one enzyme, in particular a proteolytic enzyme. Particularly suitable tapes are those, wherein said second surface of the base layer at least partially is of adhesive nature and/or wherein said second surface is at least partially covered with at least one adhesive layer and/or wherein said second surface of the first film, in particular dissolvable film, at least partially is of adhesive nature and/or wherein said first film, in particular dissolvable film, comprises at least one adhesive. That is, in one execution the tape of the present invention presents an adhesive tape.

With these tapes, in particular adhesive tapes, it is preferable that the first film, in particular dissolvable film, covers part of said base layer and/or part of said adhesive surface and/or of said adhesive layer. Preferably, the circumferential rim of the first film, in particular dissolvable film, lies within the boundaries of the base layer and/or the adhesive layer. In one embodiment, the adhesive layer or the adhesive surface of the base layer can be used to adhere the first film, in particular dissolvable film. Those parts of the adhesive layer which are not covered by the first film, in particular dissolvable film, can then be used to adhere the tape to the skin of the user.

The tapes, in particular the adhesive tapes, of the present invention in general encompass for example all known types of tapes, e.g. tapes, plasters, band aids and bandages. The tapes of the present invention are usually not subjected to any restrictions as to their size and shape. The size and shape of these tapes can be adapted to the respective usages. It is a benefit of the tapes of the present invention to be effective also when having a rather small size. In particular by such a reduction in size the application and removal of said tapes will not cause any problems for the user. Even small tapes are still highly effective, so that the tapes of the present invention can be easily applied to any location where a skin disorder shall be remedied, and can also be easily removed.

The tapes according to the present invention can be used, for example, for the treatment of skin disorders such as dry skin, dandruff, acne, keratoses, psoriasis, eczema, skin flakiness, pruritus, age spots, lentigines, melasmas, wrinkles, warts, blemished skin, hyperpigmented skin, hyperkeratotic skin, inflammatory dermatoses, and hyperkeratotic skin such as corns and calluses.

Suitable enzymes include, for example, proteases, also called proteolytic enzymes. Proteases and proteolytic enzymes in the meaning of the present invention include peptidases. Keratinases are preferred proteases in the meaning of the present invention. Suitable proteases also encompass acidic proteases. Such acidic proteases can be taken from fungal proteases, bacterial proteases, mammalian proteases and mixtures thereof. Suitable peptidases include aminopeptidases as well as trypsin. Among suitable proteases subtilisin is preferred. Proteases are commercially available, for example, under the trade name Alkalaze ® from the company Novozymes. Suitable aminopeptidases are commercially available under the trade name Fla-vourzymes® from the company Novozymes. Also mixtures of different enzymes, e.g. as exemplified above, can be used with the dissolvable layer.

The enzymes are usually present in and/or on the first film, in particular dissolvable film, in the range from about 0,1 to about 20 weight-%, preferably in the range from about 0,5 to about 10 weight-%, and more preferably in the range from about 1,5 to about 6 weight-%, based on the weight of the first film, in particular dissolvable film,. The amounts of the enzyme are based on purified dried enzyme. Usually, it is already sufficient to employ not more than about 4 weight-%, in particular not more than about 2.5 weight-% of enzyme, based on the weight of the first film, in particular dissolvable film. Preferably, the enzyme(s) is/are distributed, in particular homogenously distributed, in the first film, in particular dissolvable film. The first film, in particular dissolvable film, can be provided with one or more enzymes by use of methods known to the person skilled in the art.

According to another embodiment the base layer and/or the adhesive layer and/or the first film, in particular dissolvable film, can be transparent or translucent. Further, in another embodiment the base layer and/or the adhesive layer and/or the first film, in particular dissolvable film, comprise(s) hydrocolloids. Suitable hydrocolloids can be selected from naturally occurring hydrocolloids, semi-synthetic hydrocolloids and synthetic hydrocolloids. In one embodiment hydrocolloids can be selected from among cellulose derivatives such as carboxymethylcellulose (CMC), hydroxyethylcellulose and methylcellulose, pectine, gelatine, guar gum, chitosan, caraya, locust bean gum, carageenan, xanthan, collagens, dextrins, sodium or calcium alginate and/or sodium starch glycolate and/or synthetic polymers like polyacrylic acid, polyvinyl alcohol/acetate, polyhydroxyalkylacrylates and methacrylates, polyacrylamides, polystyrene sulfonates, polyvinyl pyrolidone, polyglycols, e.g. polyethylene glycol, copolymers, grafts of such copolymers or mixtures thereof. It is possible to use a combination of two or more hydrocolloids. Suitable hydrocolloids in the form of particles can have an average particle size of about 60 to 100 µm when in form of a dry powder. Finer particles can be obtained by milling or sifting. These hydrocolloid particles can for example be prepared from hydrophilic linear or cross-linked polymers, for example as outlined above. Suitable hydrocolloids and hydrocolloid particles are, for example, described in WO 2005/051333 A1.

According to one embodiment of the present invention, the first film also comprises a fabric, non-woven sheet or pad or a, in particular, polymeric foam or film, e.g. polyurethane foam. Suitable embodiments of the first film therefore also comprise an adhesive polyurethane film into which for example hydrocolloid particles are embedded.

The base layer preferably comprises or is made from polymeric material, in particular in the form of a polymeric film, or comprises or is made from fabric, net or nonwoven sheet or pad, or comprises or is made from a metal film, in particular an aluminium film, and can optionally also contain an adhesive. A suitable embodiment of such a base layer comprises a polyester film, a polyolefin film, in particular a polyethylene film, a polyurethane film or a PVC film. A suitable nonwoven fabric for the base layer can for example be obtained from a mixture of styrenic elastomers and polyolefins as described in JP 07024049 A.

Dissolvable films in the meaning of the present invention shall comprise those films which are at least partially dissolvable or disintegratable and which at least partially dissolve or disintegrate when in contact with polar substances such as water or bodily fluids, e.g. sweat or other bodily secretions. Thus, the dissolvable film in the meaning of the present invention is dissolvable in a hydrophilic environment. A dissolvable film in the meaning of the present invention therefore also encompasses a disintegratable film. For example, in one embodiment the dissolvable film can be a water soluble film. For a dissolvable film in the meaning of the present invention it is sufficient that upon dissolution or disintegration the enzyme contained therein is released. The dissolvable or disintegratable film can be prepared by methods known to the person skilled in the art. The film components can for example be added and formed into a solution or dispersion. Alternatively, the film can also be produced by a dry blend. From said solutions or dispersions the film can be obtained by known processing steps such as casting, drawing or extruding techniques. The solution or dispersion can for example, be sprayed or roll coated. The dry blend may be extruded to form a film. The first film, in particular dissolvable film, in the meaning of the present invention can be mono- or multilayered films.

In a preferred embodiment the dissolvable film comprises at least one cellulose derivative. Among these cellulose derivatives hydroxyalkyl cellulose such as hydroxypropyl cellulose and/or hydroxypropyl methylcellulose are particularly preferred. The dissolvable film in particular when comprising a cellulosic derivate such as hydroxypropyl cellulose and/or hydroxypropyl methylcellulose also contains fatty acids or fatty acid derivatives or salts or esters thereof and/or surfactants such as anionic surfactants. Suitable fatty acids or fatty acid derivatives or salts or esters thereof comprise for example those having a C₁₄₋₁₈ backbone.

It is also possible according to another aspect of the invention that the dissolvable film, in particular in addition to any of the aforementioned cellulose derivatives, comprises at least one plasticiser, in particular sorbitol, polyethylene glycol and/or polyethylene oxide, and/or at least one starch component, and/or at least one polysaccharide, and/or at least one filler and/or at least one humectant and/or polyvinyl alcohol.

Besides one or more enzymes the dissolvable film can in one embodiment also contain stabilizers, preservatives, glycerol, propylene glycol and/or sorbitol.

In a particularly suitable embodiment the dissolvable film of the tape of the present invention comprises a first water soluble component, in particular in the form of any of the aforementioned cellulose derivatives such as hydroxypropyl cellulose and/or hydroxypropyl methylcellulose, having a molecular weight of about 5.000 Daltons to about 60.000 Daltons and a second water soluble component, in particular in the form of any of the aforementioned cellulose derivatives such as hydroxypropyl cellulose and/or hydroxypropyl methylcellulose, having a molecular weight greater than about 60.000 Daltons. Said first water soluble component and said second water soluble component preferably comprise hydroxypropyl methylcellulose.

The dissolvable films of the present invention can be made according to known processes as, for example, described in US 2008/0124381 A1, WO 2009/048924 A1 and WO 2005/040228 A2. Usually, prior to the formation of the dissolvable film the enzymes and, if need be, any of the above described additives can be added to the components forming said dissolvable film. It is preferred that purified enzymes are incorporated into the first film, in particular dissolvable film. How to obtain purified enzyme is well known by the person skilled in the art. According to a suitable procedure, enzymes, in particular purified enzymes, e.g. obtained by way of lyophilisation and/or chromatography, are used in the preparation of the dissolvable films in the form of aqueous eluates. These eluates can, for example, contain from about 5 to about 50 weight-% enzyme, in particular purified enzyme. It is also possible to add formulation agents, such as glycerol, propylene glycol and/or sorbitol stabilisers and/or preservatives to said eluates.

It also preferred to employ hydrocolloids with the first films, in particular the dissolvable films, for example in the form of hydrocolloid particles, e.g. as described above for the base layer, adhesive layer and dissolvable film.

In one embodiment satisfying results have been obtained by incorporating at least one hydrocolloid together with at least one enzyme in the first film, in particular the dissolvable film.

The adhesives used to render the second surface of the base layer at least partially tacky or adhesive or to prepare the adhesive layer can according to one embodiment be selected from the group consisting of polyurethane adhesives, hotmelt adhesives, acrylic adhesives, and hydrocolloid adhesives, in particular pressure sensitive hydrocolloid adhesives. Such adhesives are known to the person skilled in the art and are also commercially available. Suitable adhesive layers can for example be made from polyurethanes, polyesters, porous polyolefins and porous polyvinylchloride. According to another embodiment the adhesive material used for rendering the second surface tacky or for forming the adhesive layer can comprise a rubbery continuous phase having a discontinuous phase distributed therein which comprises a cyclodextrin, in particular in an amount of 0.1 to 65 weight-%, and optionally a hydrocolloid other than cyclodextrin. The continuous phase can comprise for example polyisobutylene or a rubbery co-polymer of styrene. Such adhesive systems can also comprise trans-polyoctenamers as, for example, disclosed in WO 2001/30406 A1. Suitable acrylic adhesives comprise, among others, water-soluble or water-dispersable draft co-polymers of at least one water-soluble base polymer such as hydroxyethylacrylate and a hydrophilic macromer such as an ethoxylated or propoxylated hydroxyalkyl (meth)acrylate. Acrylic adhesives can also comprise a blend of a copolymer of an alkyl(meth)acrylate monomer having a Tg > 20°C, a C₁₋₃₀(meth)acrylate monomer, an nitrogen-containing polar monomer and a polymerizable epoxy-containing monomer as for example disclosed in WO 2002/100969 A1. The adhesive layer according to another embodiment can comprise a porous polymer film selected from the group consisting of polyacrylates, polyurethanes, polybutadiene, polyisoprene, polyesters, silicone polymer poly(alkylene oxides) and polyisobutylene.

In one embodiment it is provided that the base layer or the adhesive layer have an average thickness in the range from about 5 µm to about 2 mm, in particular from about 0,5 mm to about 1,5 mm. The first film, in particular dissolvable film, usually has one average thickness in the range from about 5 µm to about 2 mm, preferably from about 10 µm to about 1 mm, and also preferably from 10 µm to about 0,2 mm. As one advantage of the present invention it has been found that a rather thin first film, in particular dissolvable film, can be used without having to encounter any decrease in its effect on the skin. In particular due to being able to use very thin first film, in particular dissolvable film, the thickness of the entire tape according to the invention can be kept rather small thereby making its use even more convenient. The tapes of the present invention can be made that thin that they will not be perceived at all during use by the wearer. In one embodiment, the adhesive layer, which for example can be a porous hydrophilic adhesive layer, has an average thickness in the range of about 50 µm to about 80 µm. According to another, or the same, embodiment, the base layer which can be in the form of a polyurethane film, can have an average thickness of in the range from about 20 µm to about 50 µm.

The tapes, in particular the adhesive tapes, according to the present invention can also be equipped with at least one support layer which faces the first surface of the base layer. Said support layer is usually adhered to said base layer. In another embodiment one or more first intermediate layers can be present between the base layer and the support. In addition, or alternatively, on or more second intermediate layers can be present between the base layer and the first film, in particular dissolvable film,. Suitable support layers can for example comprise nonwoven sheets, polymeric films, e.g. polyurethane films, paper, in particular siliconized paper, or polymeric nets. The tapes, in particular adhesive tapes, of the present invention can further comprise a removable cover layer, in particular a release liner, on the side of the carrier layer and/or the adhesive layer.

According to another embodiment the base layer and/or the first film, in particular dissolvable film, and/or the support layer are transparent or translucent.

The present invention also provides such tapes, in particular adhesive tapes, in which the dissolvable film is coloured and/or has a colour different from the base layer and/or the adhesive layer. Insofar as the dissolvable film regularly dissolves upon contact with the skin, such a colouring can be used to control disintegration of the dissolvable film, i.e. to monitor the degree in which hyperkeratosis inhibitors and/or keratinolytic agents have been consumed. Insofar as the dissolvable film can be employed with having a very small thickness it is very practical for the user to be able to follow the action of the tapes, e.g. adhesive tapes of the present invention by way of colour change. Due to its very small thickness it is sometimes not easily discernible when all active ingredients of the dissolvable film have been consumed. Suitable colouring agents comprise for example food-grade colorants such as disclosed in US 4,400,400, e.g. colorants which are enzymatically degradable (thereby losing their colour) when being released in or from a dissolvable film.

With tapes, in particular the adhesive tapes, of the present invention it has been found that by including enzymes in the first film, in particular dissolvable film, very fast curing rates can be obtained. For example, with hyperkeratotic tissue such as corns and calluses rapid removal of the stratum corneum could be observed. It also has been found that the tapes of the present invention allow for a rather discrete usage since they can be prepared having a rather small thickness. In consumer tests it was reported that the tapes, e.g. the adhesive tapes according to the present invention behave like a second skin. The tapes, e.g. the adhesive tapes according to the present invention can be easily made water proof. In addition, these tapes do not easily role up, stay easily in place, and do not exhibit wrinkles upon use. Surprisingly, it has been found that enzymes, in particular proteolytic enzymes such as keratinases, when incorporated into the first film, in particular dissolvable film, of the tape, e.g. the adhesive tapes of the present invention remain stable and do not loose any of their activity over a long period of time. These enzymes are significantly more stable than in their usually employed aqueous systems. For example, these enzymes should be stable in the first film, in particular dissolvable film,s for more than one year, in particular for more than three years. The tape of the present invention can be stored and shipped without spending much effort in maintaining the activity of the active ingredients within the first film, in particular dissolvable film,. Moreover, it also has surprisingly been found that by incorporating enzymes into the first film, in particular dissolvable film, their activity on skin disorders can be finely adjusted.

In the following, the invention will be described by way of examples. The present invention is of course not limited in its scope by these examples which just serve the purpose to illustrate specific embodiments of the present invention and to provide means for carrying out the present invention.

The tapes tested had the following composition. The base layer was made of a polyurethane film having a basis weight in the range from 25 to 35 g/m². The adhesive layer was a pressure sensitive hydrocolloid adhesive layer and had an average thickness of about 1.2 mm. The dissolvable film was a hydroxylpropyl methylcellulose based film and had an average thickness of about 17 µm.

### Sample 1:

With a tape as described above the dissolvable film contained about 1.5 weight-% subtilisin, based on the weight of the dissolvable film. The amount of subtilisin was determined based on purified, dried enzyme.

### Sample 2 (comparative sample):

As Sample 2 a commercially available corn removal plaster was used. This plaster comprised a polymeric film as the base layer, and a foam disc with a hole in its center. This hole was filled with a paste containing about 40 weight-% salicylic acid.

The efficacy of Samples 1 and 2 was tested on skin explants on which corn-like stratum corneum formation had been induced. The skin explants had an average diameter of about 10 mm and were taken from an abdominal plasty. The explants were kept in survival in culture medium at 37°C. Samples 1 and 2 were cut the size of the explants and were then applied to said explant.

Histological analyses were carried out on explants obtained after corn-like stratum corneum formation, and obtained after one, two and four days of treatment with Samples 1 and 2. Explants obtained after either corn induction or a treatment with any of Samples 1 and 2 were collected and fixed in ordinary Bouin's solution. This fixation process in ordinary Bouin's solution took 48 hours. The samples were then dehydrated and impregnated in paraffin using a Leica 1020 dehydration automat. The samples were embedded using a Leica EG 1160 embedding station. 5-µm-thick sections were made using a Leica RM 2125 Minot-type microtome, and the sections obtained were then mounted on Superfrost® histological glass slides. Microscopical observations were made using a Leica DMLB microscope with a x40 objective. Pictures were taken with a tri CCD Olympus camera and stored using the Cell^{D} data storing software. The observation of the general morphology, in particular of the degree of removal of the stratum corneum, was performed after staining of paraffinized sections according to Masson's trichrome, Goldner variant. The measurement of the thickness of the stratum corneum was made using the Olypmus Cell^{D} software.

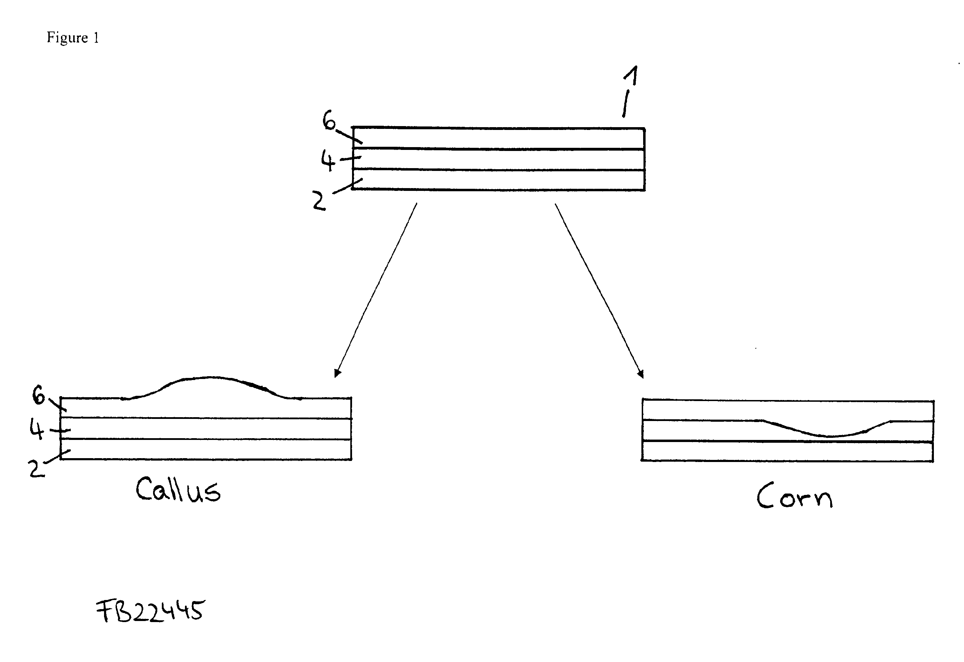

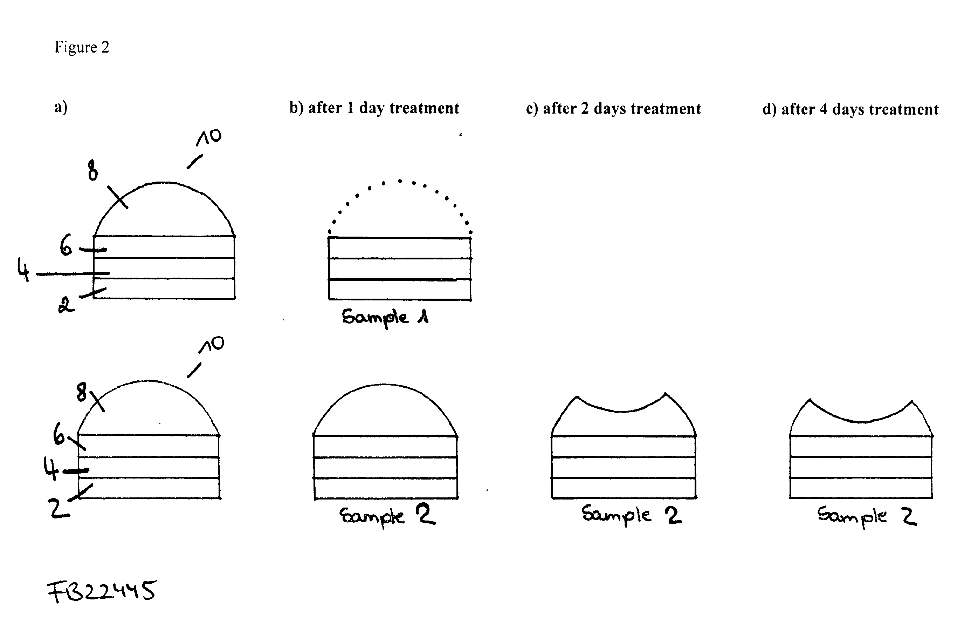

It could be shown that within one day of treatment with Sample 1 a complete and total removal of the excessive stratum corneum occurred, whereas with Sample 2 the excessive stratum corneum was only slightly removed even after four days, that is, significantly less than half of the excessive stratum corneum was removed. This result is exemplified in a schematical manner in Figure 2. To facilitate matters Figure 1 schematically exemplifies the formation of corn on the one hand and of callus on the other hand. As explained under Fig. 1 the skin 1 comprises different layers, namely the dermis 2, epidermis 4, and the stratum corneum 6. Corns and calluses are formed by enlarging the stratum corneum, e.g. due to mechanical stress. In normal skin 1 the stratum corneum 6 does not show any abnormal thickness. The stratum corneum 6 lies on the epidermis 4 which in turn lies on the dermis 2. Callus formation yields locally thickened stratum corneum 6, i.e. excessive stratum corneum formation. Corn formation, instead, usually furnishes local thickening of the stratum corneum while simultaneously the thickness of the epidermis 4 is significantly reduced. Fig. 2a shows a schematic cross-sectional view of a skin explant 10 after induced corn-formation as described above. As shown schematically in Fig. 2b) after one day of treatment with Sample 1 the entire excessive stratum corneum 6 was removed. As is also shown in Fig. 2b) to 2d) with Sample 2 excessive stratum corneum 6 was only partially removed even after four days of treatment.

Although modifications and changes maybe suggested by those skilled in the art, it is the intention of the applicant to embody within the patent warranted hereon all changes and modifications as reasonably and probably come within the scope of this contribution to the art. The features of the present invention which are believed to be novel are set forth in detail in the appended claims. The features disclosed in the description as well as the claims could be essential alone or in every combination for the realization of the invention in its different embodiments.

## Claims

1. Tape, in particular adhesive tape, for the treatment of skin disorders comprising at least one base layer having a first surface facing away from the skin during use and a second surface facing towards the skin during use, and at least one first film, in particular dissolvable film, on the side of said second surface, which covers at least part of the second surface of said base layer, said first film, in particular dissolvable film, having a first surface facing away from the skin during use and a second surface facing towards the skin during use and comprising at least one enzyme, in particular a proteolytic enzyme.

2. Tape according to claim 1 wherein said second surface of the base layer at least partially is of adhesive nature and/or wherein said second surface is at least partially covered with at least one adhesive layer and/or wherein said second surface is at least partially covered with at least one adhesive nature and/or wherein said first film, in particular dissolvable film, comprises at least one adhesive.

3. Tape according to claim 2, wherein said first film, in particular dissolvable film, covers part of said base layer and/or part of said adhesive surface and/or of said adhesive layer.

4. Tape according to any of the preceding claims, wherein the proteolytic enzyme comprises a keratinase, in particular subtilisin, or a peptidase, in particular an aminopeptidase or trypsin.

5. Tape according to any of the preceding claims, wherein the base layer and/or the adhesive layer and/or the first film, in particular dissolvable film, is/are transparent or translucent, and/or wherein the base layer and/or the adhesive layer and/or the first film, in particular dissolvable film, comprise hydrocolloids.

6. Tape according to any of the preceding claims wherein the base layer comprises or is made from polymeric material, in particular in the form of a polymeric film, fabric, net or nonwoven sheet or pad, or comprises or is made from a metal film, in particular an aluminium film, and optionally an adhesive.

7. Tape according to claim 6, wherein the base layer comprises a polyester film, a polyolefin film, in particular a polyethylene film, a polyurethane film or a PVC film.

8. Tape according to any of claims 2 to 7, wherein the adhesives are selected from the group consisting of polyurethane adhesives, hot-melt adhesives, acrylic adhesives, and hydrocolloid adhesives, in particular pressure sensitive hydrocolloid adhesives.

9. Tape according to any of the preceding claims, wherein the dissolvable film comprises at least one cellulose derivative, preferably hydroxyalkyl cellulose, more preferably hydroxypropyl cellulose and/or hydroxypropyl methylcellulose.

10. Tape according to any of the preceding claims, wherein the dissolvable film comprises at least one plasticiser, in particular sorbitol, polyethylene glycol and/or polyethylene oxide, and/or at least one starch component, and/or at least one polysaccharide and/or at least one filler and/or at least one humectants and/or polyvinyl alcohol.

11. Tape according to any of the preceding claims, wherein the dissolvable film comprises a first water soluble component, preferably hydroxypropyl cellulose and/or hydroxypropyl methylcellulose, having a molecular weight about 5.000 Daltons to about 60.000 Daltons and a second water soluble component, preferably hydroxypropyl cellulose and/or hydroxypropyl methylcellulose, having a molecular weight greater than about 60.000 Daltons.

12. Tape according to any of the preceding claims, wherein the base layer or the adhesive layer have an average thickness in the range from about 5 µm to about 2 mm, in particular from about 0,5 mm to about 1,5 mm, and/or wherein the first film, in particular dissolvable film, has an average thickness in the range from about 5 µm to about 2 mm, preferably from about 10 µm to about 1 mm.

13. Tape according to any of the preceding claims further comprising at least one support layer facing the first surface of the base layer, in particular in the form of a nonwowen sheet or pad, a polymeric net, a foam or a fabric, and/or further comprising a removable cover layer, in particular a release liner, on the side of the first film, in particular dissolvable film, and/or the adhesive layer.

14. Tape according to any of the preceding claims, wherein the dissolvable film is coloured and/or has a colour different from the base layer and/or the adhesive layer.

15. Use of at least one first film, in particular dissolvable film, containing at least one enzyme, in particular a proteolytic enzyme, for the preparation of a tape, in particular adhesive tape, for the treatment of skin disorders, in particular according to any of the proceeding claims.

16. Use of a tape according to any of claims 1 to 14 or of a first film, in particular dissolvable film, containing at least one enzyme, in particular a proteolytic enzyme, as described in any of claims 1 to 14 to prevent and/or to reduce skin disorders, in particular the formation of hyperkeratosis and/or to degrade keratinolytic tissue.
